# EUROPEAN PATENT SPECIFICATION

(11) **EP 3 193 996 B1**
(45) Date of publication and mention of the grant of the patent: **06.03.2019**
(21) Application number: 15750757.5
(22) Date of filing: 18.08.2015
(51) Int. Cl.: A61M 16/10, B01D 53/04

(54) **DEVICE FOR PROVIDING SUPPLEMENTAL OXYGEN TO A SUBJECT**
VORRICHTUNG ZUR BEREITSTELLUNG VON ZUSÄTZLICHEM SAUERSTOFF FÜR EINE PERSON
DISPOSITIF POUR FOURNIR DE L'OXYGÈNE D'APPOINT À UN SUJET

(30) Priority: 19.09.2014 EP 14185468
(43) Date of publication of application: 26.07.2017
(73) Proprietor: Koninklijke Philips N.V., 5656 AE Eindhoven (NL)
(72) Inventor: VINK, Teunis, Johannes, NL-5656 AE Eindhoven (NL); HILBIG, Rainer, NL-5656 AE Eindhoven (NL); KOERBER, Achim, Gerhard, Rolf, NL-5656 AE Eindhoven (NL); MATHEW, Denny, NL-5656 AE Eindhoven (NL); VAN RUTTEN, Edwin, NL-5656 AE Eindhoven (NL); KELLY, Juliana Pauline, NL-5656 AE Eindhoven (NL)
(74) Representative: Tassignon, Tom
(86) International application number: PCT/EP2015/068899
(87) International publication number: WO 2016/041718

(56) References cited:
- EP-A1- 1 568 391
- EP-A2- 1 967 224
- EP-A2- 2 093 188
- WO-A1-00/12197
- WO-A1-2009/105627
- WO-A1-2013/126285
- WO-A1-2013/179173
- WO-A2-01/76671
- WO-A2-2010/129329
- US-A1- 2008 053 310
- US-A1- 2012 055 483
- US-A1- 2013 216 627

## Description

### TECHNICAL FIELD OF THE INVENTION

The invention relates to a device for providing supplemental oxygen to a subject.

### BACKGROUND TO THE INVENTION

Supplementary oxygen is a common therapy used in subjects with chronic obstructive pulmonary disease (COPD), the occurrence of chronic bronchitis or emphysema, which are common long term effects of smoking. Subjects with COPD may require additional oxygen to breathe either during a temporary worsening of their condition, or throughout the day and night. It is indicated in subjects with a partial pressure of oxygen (PaO2) equal to or less than 55 mmHg or an arterial blood oxygen saturation level (SaO2) equal to or less than 88%.

The oxygen can be delivered through a number of devices dependent on the situation, flow requirements and patient preferences. Electrically powered oxygen concentrators are most commonly used for home oxygen therapy and portable oxygen, with the advantage of having continuous supply without the need for additional deliveries of bulky gas cylinders. Oxygen concentrators operate to continuously generate and supply oxygen-enriched air (air with an oxygen content that is higher than atmospheric, e.g. 21%) to the subject.

In these devices, a nasal cannula is commonly used as a subject interface. A nasal cannula consists of a lightweight tube which on one end splits into two prongs which are placed in the nostrils and from which oxygen flows. The other end is connected to the oxygen supply such as a portable oxygen concentrator (POC).

A known solution for oxygen therapy is disclosed in US 2008/0053310. Said document discloses a portable oxygen concentrator that includes a plurality of sieve beds for adsorbing nitrogen from air, the sieve beds comprising air inlet/outlet ends and oxygen inlet/outlet ends, at least one reservoir communicating with the oxygen inlet/outlet ends of the sieve beds for storing oxygen exiting from the oxygen inlet/outlet ends of the sieve beds, a compressor for delivering air at one or more desired pressures to the air inlet/outlet ends of the sieve beds, the compressor comprising a motor coupled to a crankshaft defining a central axis, the compressor includes three heads spaced apart around the central axis and rods extending between respective heads and the crankshaft.

WO00/12197 describes a portable breathing air supply apparatus which uses a membrane separation module to provide oxygen enriched air from ambient air. The enriched air is stored in a reservoir while the user exhales. Conserver valve is in a tube leading to the user's mouth or nose and opens to discharge enriched air from the reservoir. A sensor in the tube detects the start of inhalation and triggers the conserver valve to open for a preselected duration. A fan blows the ambient air through feed pipe to the membrane module and a vacuum pump draws on the permeate side to fill reservoir with the oxygen enriched air. The apparatus can be made compact to fit into a carrying case or strapped to the body of the user and can be powered by batteries. The apparatus frees the user to roam for long periods of time away from a primary source of oxygen with little inconvenience.

WO2013/179173 describes a portable handheld pressure support system configured to deliver a blending gas enriched pressurized flow of breathable gas to the airway of a subject. The pressure support system is configured to treat patients suffering from dyspnea and/or other conditions. The therapy provided to dyspnea patients is configured to be used as needed by a subject to rapidly alleviate shortness of breath. The pressure support system is configured to be small and lightweight so that the subject may carry the system and use the system as needed without requiring a device to be worn on the face. In some embodiments, the system comprises one or more of a pressure generator, a subject interface, a blending gas inlet port, one or more sensors, a valve, one or more processors, a user interface, electronic storage, a portable power source, a housing, a handle, and/or other components.

WO2010/129329 describes an oxygen concentrator apparatus, as well as methods incorporating use of the apparatus. The apparatus and methods utilize selected cycle times, adsorbent specifications and novel conditions to produce a fast Pressure Swing Adsorption ("PSA") system. The oxygen concentrator apparatus and methods herein have significant utility in the fields of biotechnology, engineering, and medicine.

WO01/76671 describes an apparatus and method for performing positive pressure (PP) therapy alone or in combination with an aerosol delivery apparatus. The positive pressure apparatus includes a positive pressure valve having a continuously variable respiratory window. The PP valve may be associated with a patient respiratory system interface alone, such as, but not limited to, a mask or mouthpiece, or in combination with an aerosol delivery apparatus.

WO2013/126285 describes an oxygen concentrator system which includes a portable unit and a base unit. The portable unit includes an air separation device. The base unit includes a vacuum pump. The portable unit is moveable with respect to the base unit between a connected position, in which the vacuum pump of the base unit is connected to the air separation device of the portable unit for applying vacuum pressure at the air separation device of the portable unit, and a disconnected position, in which the vacuum pump is not connected to the air separation device of the portable unit. The air separation device operates using a vacuum pressure swing adsorption (VPSA) cycle when the portable unit is in the connected position. The air separation device operates using a pressure swing adsorption (PSA) cycle when the portable unit is in the disconnected position. As examples, the portable unit can be configured to be carried as a backpack, with a shoulder strap, using a wheeled cart, or by attachment to a wheelchair or other mobility aid. The air separation device is in fluid communication with the positive pressure outlet side of a compressor.

EP1967224 describes an oxygen concentrator system including a portable system having a portable oxygen concentrator with a portable compressor. The portable system further includes a power supply, a control system, and a portable oxygen outlet configured to selectively provide a first direct fluid supply. A station is configured to engage the portable system. The station may be selectively controlled by the control system when engaged with the portable system. The station may have a stationary oxygen concentrator that is inoperable for delivering a second direct fluid supply via a stationary oxygen outlet during delivery of the first direct fluid supply via the portable oxygen outlet when the station and portable system are engaged. Further, the portable oxygen concentrator is inoperable for delivering the first direct fluid supply via the portable oxygen outlet during delivery of the second direct fluid supply via the stationary oxygen outlet when the station and portable system are engaged.

US 2013/0216627 discloses a portable device for providing oxygen-enriched air using an ultra rapid absorption cycle based on advanced molecular sieve materials.

EP 1 568 391 discloses a dual mode oxygen concentrator having a portable part and a base unit.

The current solutions used for oxygen therapy in COPD are far from ideal. The oxygen delivery devices are bulky and even the portable oxygen concentrators are not really light in weight (typically weighing 4-10 lbs/1.8-4.5 kg). Furthermore, the nasal cannula is not appreciated by its users since it is not discrete, uncomfortable in use, and most importantly it can reinforce the stigma of being perceived as a very ill person.

The above disadvantages are difficult to overcome for subjects who are in need of supplemental oxygen on a 24 hour basis. They are fully dependent on oxygen, also during the day time, and are therefore forced to use the currently available equipment. Subjects with less severe or more moderate COPD or other breathing disorders may use or be able to use supplemental oxygen in a different way, i.e. not continuously but more intermittently or on demand. These subjects can supplement their oxygen before, during and/or after certain activities and/or physical exercises. After that they can, for example, engage with their social contacts without having to use the equipment (including without having to wear the nasal cannula) and the stigma that comes with it (nasal cannula). However, these subjects also have to use the existing devices in order to provide this supplemental oxygen and boost their blood oxygenation levels. These existing devices, although designed to be portable, are not designed with this type of use in mind, and there is therefore an opportunity to provide a device for providing supplemental oxygen that is much lighter and portable than existing devices and that is able to provide supplemental oxygen over a short timescale.

### SUMMARY OF THE INVENTION

The invention is defined by the claims.

According to a first aspect, there is provided a handheld device for providing supplemental oxygen to a subject, the device comprising a subject interface through which the subject can inhale; a container that has a first outlet connected to the subject interface to allow gas with an elevated oxygen level stored in the container to be inhaled by the subject, a first inlet, and a material for removing a specific gas from air passing through the container to increase the oxygen content of the air passing through the container; and an air blower that is connected to the first inlet of the container and that is configured to supply air into the container as the subject uses the device; and a first valve located between the container and the subject interface for enabling a flow of supplemental oxygen to the subject.

In some embodiments the container is configured to store gas with an elevated oxygen level at a pressure above atmospheric pressure.

In some embodiments the air blower is configured to be activated to supply air into the container when the pressure of the gas with an elevated oxygen level stored in the container is at or close to atmospheric pressure.

In some embodiments the air blower is configured to be deactivated when any one of the following conditions is satisfied (i) when the material is saturated with the specific gas; (ii) after a predetermined time from a first inhalation by the subject; (iii) after the subject has used the device for a predetermined time from the last recharge of the device; (iv) after a predetermined number of inhalations by the subject; (v) when there is an increase in the temperature of the gas supplied to the subject; or (vi) when the oxygen content of the gas supplied to the subject is below a threshold.

In some embodiments the first inlet and the first outlet are arranged with respect to each other such that air blown into the container through the first inlet by the air blower pushes out gas with an elevated oxygen level stored in the container through the first outlet.

In some embodiments the device further comprises a delivery valve positioned between the subject interface and the first outlet, the delivery valve being configured to release gas with an elevated oxygen level from the container to the subject when the subject inhales.

In some embodiments, the device further comprises a check valve positioned between the air blower and the first inlet, the check valve being configured to close when the pressure of the gas with an elevated oxygen level stored in the container is above atmospheric pressure in order to prevent gas in the container from flowing through the air blower when the air blower is deactivated, and being configured to open when the pressure of the gas with an elevated oxygen level stored in the container is at or below atmospheric pressure in order to allow air to be supplied into the container by the air blower.

In some embodiments the device further comprises a second inlet that is configured to receive gas with an elevated oxygen level during a recharging process; and a second outlet that is configured to exhaust waste gas during the recharging process.

In some embodiments the device further comprises a load valve connected to the second inlet that is configured to be opened at the start of a recharging process to allow gas with an elevated oxygen level to be supplied into the container in order to purge the material of the specific gas; and an exhaust valve connected to the second outlet that is configured to be opened at the start of the recharging process to allow waste gas to exit the container.

In some embodiments the exhaust valve is configured to be closed during the recharging process while gas with an elevated oxygen level is supplied into the container through the load valve and second inlet in order to store gas with an elevated oxygen level in the container.

In some embodiments, the device further comprises means for applying a positive air pressure to the subject when the subject exhales through the subject interface.

In some embodiments the device further comprises a control unit that is configured to control the operation of the device.

In some embodiments the specific gas is nitrogen. In some embodiments the material is an adsorbent material for adsorbing the specific gas. In some embodiments the adsorbent material is a zeolite. In other embodiments the material is an absorbent material for absorbing the specific gas.

According to a second aspect, there is provided a supplemental oxygen system comprising a handheld device as described above; and a source of gas with an elevated oxygen level that can be selectively coupled to the device in order to recharge the device with gas with an elevated oxygen level and to purge the material of the specific gas.

In some embodiments the source of gas with an elevated oxygen level is an oxygen concentrator.

In some embodiments the source of gas with an elevated oxygen level is part of a base unit for the device.

There is provided a method of providing supplemental oxygen to a subject, which is not part of the present invention, the method comprising providing a handheld device that comprises a subject interface through which the subject can inhale; a container that has a first outlet connected to the subject interface to allow gas with an elevated oxygen level stored in the container to be inhaled by the subject, a first inlet, and a material for removing a specific gas from air passing through the container to increase the oxygen content of the air passing through the container; an air blower that is connected to the first inlet of the container and that is configured to supply air into the container; and a valve for controlling the flow of supplied air, said valve being positioned between the air blower and the first inlet; dispensing gas with an elevated oxygen level to the subject through the first outlet and subject interface; and activating the air blower to supply air into the container during use of the device by the subject.

In some examples prior to the step of dispensing, the container stores gas with an elevated oxygen level at a pressure above atmospheric pressure, and the step of activating the air blower is performed when the pressure of the gas with an elevated oxygen level stored in the container is at or close to atmospheric pressure.

In some examples the method further comprises the step of deactivating the air blower.

In some examples the step of deactivating the air blower is performed when any one of the following conditions is satisfied (i) when the material is saturated with the specific gas; (ii) after a predetermined time from a first inhalation by the subject; (iii) after the subject has used the device for a predetermined time from the last recharge of the device; (iv) after a predetermined number of inhalations by the subject; (v) when there is an increase in the temperature of the gas supplied to the subject; or (vi) when the oxygen content of the gas supplied to the subject is below a threshold.

In some examples the step of activating the blower is performed in order to push out gas with an elevated oxygen level stored in the container through the first outlet to the subject interface.

In some examples the step of dispensing comprises dispensing gas with an elevated oxygen level from the container to the subject when the subject inhales.

In some examples the method further comprises the step of recharging the device with gas with an elevated oxygen level.

In some examples the step of recharging the device comprises connecting a second inlet of the container to a source of gas with an elevated oxygen level; supplying gas with an elevated oxygen level from the source through the container to purge the material of the specific gas; and allowing the purged specific gas to exit the container through an outlet of the container.

In some examples the step of recharging the device further comprises preventing gas from exiting the container once the material has been purged of the specific gas so that gas with an elevated oxygen level from the source is stored in the container.

In some examples the step of recharging the device further comprises continuing to supply gas with an elevated oxygen level into the container until the gas with an elevated oxygen level stored in the container is at a required pressure.

In some embodiments the specific gas is nitrogen. In some embodiments the material is an adsorbent material for adsorbing the specific gas. In some embodiments the adsorbent material is a zeolite. In other embodiments the material is an absorbent material for absorbing the specific gas.

According to a third aspect, there is provided a handheld device for providing supplemental oxygen to a subject, the device comprising a subject interface through which a subject can inhale; a container for storing gas with an elevated oxygen level that is connected to the subject interface so as to allow stored gas with an elevated oxygen level to be inhaled by the subject; and means for applying a positive air pressure to the subject when the subject exhales through the subject interface.

Various embodiments of the third aspect are also contemplated in which the device is configured according to any of the above embodiments of the first aspect.

### BRIEF DESCRIPTION OF THE DRAWINGS

For a better understanding of the invention, and to show more clearly how it may be carried into effect, reference will now be made, by way of example only, to the accompanying drawings, in which:
Figure 1 schematically shows an exemplary supplemental oxygen system;
Figure 2 is a schematic diagram of a handheld device according to an aspect of the invention;
Figure 3 schematically shows the handheld device of Figure 2 when oxygen is being dispensed to the subject;
Figure 4 is a graph illustrating the operation of the valves in the handheld device when oxygen is being dispensed to the subject;
Figure 5 is a flow chart illustrating the method of operating the handheld device to dispense oxygen to the subject, which is not part of the present invention;
Figure 6 illustrates an exemplary nitrogen concentration profile during the operation of the handheld device;
Figure 7 schematically shows the handheld device of Figure 2 when the device is being recharged;
Figure 8 is a graph illustrating the operation of the valves in the handheld device when the device is being recharged;
Figure 9 is a flow chart illustrating the method of recharging the handheld device, which is not part of the present invention;
Figure 10 illustrates an exemplary nitrogen concentration profile during the recharging of the handheld device; and
Figure 11 is a schematic diagram of a handheld device according to an aspect of the invention.

### DETAILED DESCRIPTION OF THE PREFERRED EMBODIMENTS

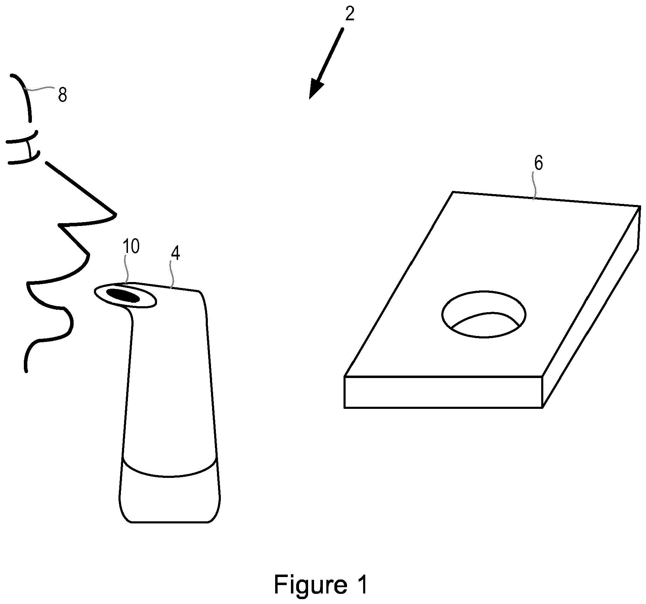

Figure 1 shows an embodiment of the supplemental oxygen system according to an aspect of the invention. In this embodiment, the system 2 comprises a handheld device 4 and a base unit 6 for the handheld device 4. The handheld device 4 is configured to deliver supplemental oxygen (otherwise interchangeably referred to herein as "gas with an elevated oxygen level" (i.e. elevated above that normally found in air), "oxygen-enriched air", "oxygen-enriched gas", "gas enriched with oxygen" or "enriched oxygen-comprising gas") to a user or subject 8 on demand. The handheld device 4 is designed to be easily held by the subject 8, and is able to hold and/or supply supplemental oxygen into a number of breaths by the subject 8 through the handheld device 4. The base unit 6 is provided to recharge or replenish the oxygen level of the handheld device 4 when the handheld device 4 is coupled to the base unit 6. The base unit 6 may comprise an oxygen concentrator, for example an oxygen concentrator that operates according to the known pressure swing adsorption cycle principle, that supplies air or gas with elevated levels of oxygen (i.e. elevated above the normal oxygen content of air at sea level of around 21%) to the handheld device 4. Alternatively, the base unit 6 may contain or be otherwise coupled to a source of liquid oxygen or compressed air or gas having an elevated oxygen level that can be used to recharge or replenish the handheld unit 4. The base unit 6 may also be configured to recharge a battery inside the handheld unit 4.

In alternative embodiments, the system 2 may only be comprised of a handheld device 4; no separate base unit is required for recharging or replenishing the oxygen level of the handheld device 4. In these embodiments, the handheld device 4 may be replenished with oxygen by replacing a removable oxygen container in the handheld device 4 or by connecting the handheld device 4 to a supply of oxygen or gas with an elevated oxygen level (for example a cylinder containing liquid oxygen or a source of compressed air having elevated oxygen levels).

In all of the above embodiments, the handheld device 4 is provided with a subject interface 10 through which the subject 8 is to inhale in order to receive the supplemental oxygen from the handheld device 4. In some embodiments, the subject 8 may also be able to (or required to) exhale through the subject interface 10.

The subject interface 10 is preferably a mouthpiece, but other types of interface that can be non-invasively engaged by the mouth and/or nose of the subject 8 can be used. Suitable interfaces include, but are not limited to, a nasal cannula, a nasal mask, a nasal oral mask, a full face mask, and a total face mask.

The supplemental oxygen system 2 can be for use by subjects with chronic obstructive pulmonary disease (COPD) or other breathing disorders where additional oxygen (e.g. at or above levels of 20%-40%) is required or beneficial to the subject 8.

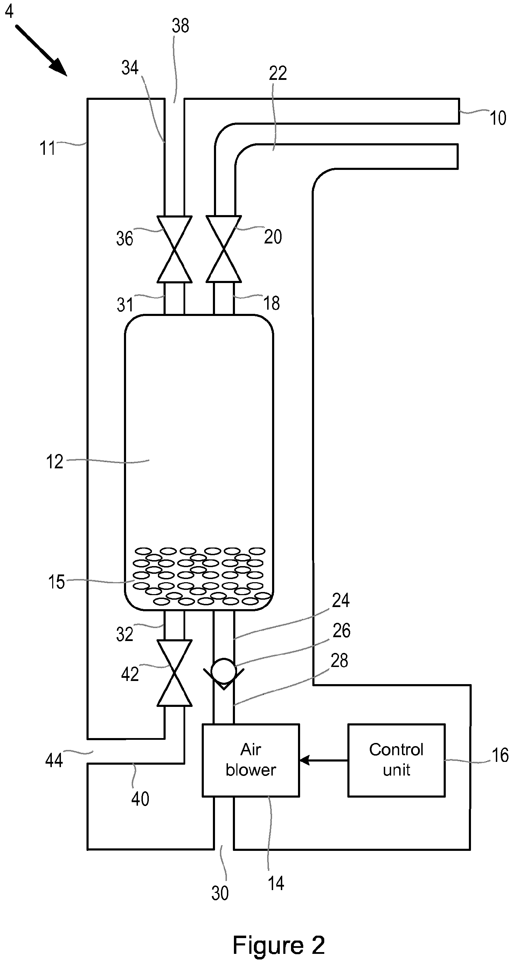

Figure 2 is a schematic diagram of a handheld device 4 according to an embodiment of the invention. Preferably, the handheld device 4 is sufficiently small and lightweight to make it readily portable for a subject 8 while still providing a useful number of breaths with supplemental oxygen, and the components of the handheld device 4 are designed and/or selected accordingly.

The handheld device 4 comprises a subject interface 10 as described above, a housing 11, a container or tank 12 that stores and generates the oxygen to be dispensed to the subject 8 when the subject 8 inhales through the subject interface 10, an air blower, air pump or fan 14 that is arranged to supply air into the container 12 and a control unit 16 that controls the operation of the handheld device 4, including the operation of the air blower 14.

The housing 11 may be shaped, contoured and/or textured to enable it to be easily held by the subject 8.

The container 12 contains a material 15 for removing one or more specific gases from the air or other gas present in or passing through the container 12. The material 15 is provided to remove the one or more specific gases from the gas in the container 12 in order to increase the concentration of oxygen above that found in normal air (e.g. around 21% at sea level). In preferred embodiments, the material 15 removes nitrogen from the gas in the container 12. In preferred embodiments, the material 15 is an adsorbent material that adsorbs a specific gas or gases (e.g. nitrogen) from the air or other gas present in the container 12. In these embodiments, the adsorbent material 15 is preferably a zeolite, such as a Li-doped Low-Silica zeolite X (LiLSX) zeolite. In alternative embodiments, the material 15 can be an absorbent material that absorbs a specific gas or gases (e.g. nitrogen) from the air or other gas present in the container 12. Such an absorbent material can comprise a metal organic framework (MOF).

Although in the following the invention is explained with reference to the material 15 being an adsorbent material, it will be appreciated by those skilled in the art that these embodiments are merely exemplary, and the invention as described could instead be implemented with an absorbent material.

In an exemplary embodiment, the container 12 has a generally cylindrical shape and a length of around 0.11 meters and a diameter of around 0.04 meters. It will be appreciated however, that containers 12 of other sizes and/or shapes can be used according to the desired size and/or shape of the handheld device 4. In this exemplary embodiment, the container 12 contains about 0.094 kg of LiLSX pellets (e.g. pellets of 0.00055 meters diameter), although it will be appreciated that other quantities of zeolites, other zeolites and/or other adsorbent materials 15 can be used.

The air blower, air pump or fan 14 is any type of device suitable for supplying air into the container 12 and provide a small increase in air pressure (overpressure) in the container 12. The terms air blower, air pump and fan are used interchangeably herein and a reference to one includes the others, and vice versa, unless otherwise expressly provided. Preferably the air blower 14 is able to provide a small overpressure in the region of, for example 0.01-0.15 bar (approximately 100-1500 mmH₂O). Preferably the maximum air flow rate of the air blower 14 in operation is between 0.3 and 1 liter/minute. In some embodiments, it is sufficient for the air blower, air pump or fan to provide an increase in pressure in the container 12 of the order of up to 10%, or up to 20% in the pressure (an air pressure rise of up to 1136 mmH₂O or up to 2066 mmH₂O respectively), although air blowers, air pumps or fans 14 that provide higher pressure rises are also contemplated. It will be appreciated therefore that the air blower 14 is preferably a small, low-power component that is suitable for use in a handheld device 4. It will also be appreciated that the air blower 14 is much smaller and less powerful than an air compressor that is often found in oxygen concentrators operating according to the pressure swing adsorption cycle.

The control unit 16 can comprise one or more processors, processing units, processing modules and/or other electronic circuitry that is configured or programmed to control the operation of the handheld device 4 as described in more detail below.

A first outlet 18 of the container 12 is connected to the subject interface 10 via a first valve 20, also referred to as the delivery valve herein, and a first conduit 22. The first valve 20 is used to release gas with an elevated oxygen level from the container 12 into the first conduit 22 and to the subject interface 10 when the subject 8 inhales. In some embodiments, the opening and closing of the first valve 20 is controlled by control unit 16. In some embodiments, the control unit 16 controls the opening and closing of the first valve 20 in response to detecting the subject 8 is inhaling or exhaling respectively. In other embodiments, the opening and closing of the first valve 20 is directly in response to the subject 8 inhaling and exhaling through the subject interface 10 respectively (e.g. the first valve 20 can be configured such that a reduction in pressure in the first conduit 22 as the patient inhales is sufficient to open the first valve 20 and either the increase in pressure in the first conduit 22 as the patient exhales is sufficient to close the first valve 20 or the first valve 20 is loaded so that it automatically closes when the patient stops inhaling). In some embodiments, the first valve 20 is a pulse dosing valve which only allows a preset volume of gas through (i.e. from the container 12) each time that the valve 20 is opened. The preset volume of gas that the first valve 20 is configured to dispense during each operation can depend on the needs of the subject 8. A typical volume is in the range of 15ml to 100ml, and in this case as normal tidal breathing volumes are of the order of 400ml to 500ml, the subject interface 10 and/or first conduit 22 can facilitate the mixing of the oxygen enriched gas dispensed through the first valve 20 with air drawn from the atmosphere to meet the required tidal volume. The construction and operation of the pulse dosing valve 20 is known to those skilled in the art, and further details are not provided herein.

The air blower 14 is connected to a first inlet 24 of the container 12 via a second valve 26 and a second conduit 28. The air blower 14 also has an air inlet 30 through which the air blower 14 can draw air into the device 4. The second valve 26 is also referred to as a check valve herein. The second valve 26 is provided to prevent gas from flowing out of the container 12 and through the air blower 14 (particularly when the air blower 14 is not active). Preferably the second valve 26 is a check valve, otherwise known as a one-way valve or non-return valve. The second valve 26 preferably operates in response to the relative pressure between the container 12 and environment, i.e. the valve 26 is closed when the gas pressure in the container 12 is higher than the air pressure external to the device 4, and the valve 26 is open when the external pressure (which can include the small overpressure caused by the operation of the air blower 14) exceeds the gas pressure in the container 12. Although a check valve 26 that operates according to the relative pressures is the preferred form for valve 26, it will be appreciated that other types of valve could be used, and it will also be appreciated that the opening and closing of the second valve 26 can be controlled by the control unit 16 (in which case the control unit 16 monitors the pressure inside the container 12 to determine when to open and close the second valve 26.

The first inlet 24 and first outlet 18 are arranged on the container 12 so as to cause air blown into the container 12 by the air blower 14 to push out the gas stored in the container 12. In some embodiments, the first inlet 24 is on a side of the container 12 that is different to the side of the container 12 having the first outlet 18. In preferred embodiments, the first inlet 24 and first outlet 18 are on opposite sides of the container 12.

To enable the container 12 to be recharged or replenished after the oxygen-enriched air in the container 12 has been depleted or dispensed (e.g. when the adsorbent material 15 is saturated with the adsorbed gas), the container 12 is provided with a second inlet 31 for receiving a 'purge' gas and a second outlet 32 for exhausting waste gas (i.e. the gas adsorbed by the adsorbent material 15). The second inlet 31 and second outlet 32 are arranged on the container 12 so as to cause oxygen-enriched air that passes into the container 12 through the second inlet 31 to push out the gas adsorbed in the adsorbent material 15. In some embodiments, the second inlet 31 is on a side of the container 12 that is different to the side of the container 12 having the second outlet 32. In preferred embodiments, the second inlet 31 and second outlet 32 are on opposite sides of the container 12 (although not necessarily on the same sides respectively as first outlet 18 and first inlet 24 as shown in Figure 2).

The second inlet 31 is connected to a third conduit 34 via a third valve 36 which leads to a supply port 38 on the outside of the handheld device 4. The third valve 36 is also referred to herein as the load valve. The supply port 38 is configured to allow a source of oxygen, oxygen-enriched air, or other purging gas to be coupled to the handheld device 4 and thus supplied to the container 12 when the load valve 36 is open. In embodiments where the handheld device 4 can be docked with a base unit 6 for recharging, the supply port 38 will be configured to connect to a corresponding port or outlet on the base unit 6 to receive the oxygen, oxygen-enriched air, or other purging gas. The operation of the third valve 36 is preferably controlled by the control unit 16.

The second outlet 32 is connected to a fourth conduit 40 via a fourth valve 42 which leads to an exhaust port 44 on the outside of the handheld device 4. The fourth valve 42 is also referred to herein as the exhaust valve. The exhaust valve 42 is opened to allow waste gas (e.g. the gas that was adsorbed by adsorbent material 15 during previous operation of the handheld device 4) to flow out of the device 4 and into the atmosphere. The operation of the fourth valve 42 is preferably controlled by the control unit 16.

Unless otherwise provided herein, any of the first valve 20, second valve 26, third valve 36 and fourth valve 42, can comprise any of the following types of valve: a plug valve, a ball valve, a check valve, a butterfly valve, a solenoid, a pressure switch, and/or other pressure regulating device.

It will be appreciated that Figure 2 only shows the components of the handheld device 4 required to illustrate this embodiment of the invention, and a practical implementation of a handheld device 4 according to the invention may include other components to those shown. For example, a handheld device 4 may comprise a power source for powering the components described above and/or a user interface for allowing the subject to control the device 4. The power source may comprise, for example, one or more replaceable or rechargeable batteries that are capable of powering the handheld device 4 for at least several minutes. The user interface comprises components for enabling the subject 8 to receive information from the device 4 and/or interact with or control the device 4, and may include, for example, one or more buttons, switches or controls for activating, deactivating and otherwise controlling the operation of the device 4, one or more displays, lights, indicators, etc. for providing a visual output to the subject 8 and/or one or more speakers for providing an audible output to the subject 8.

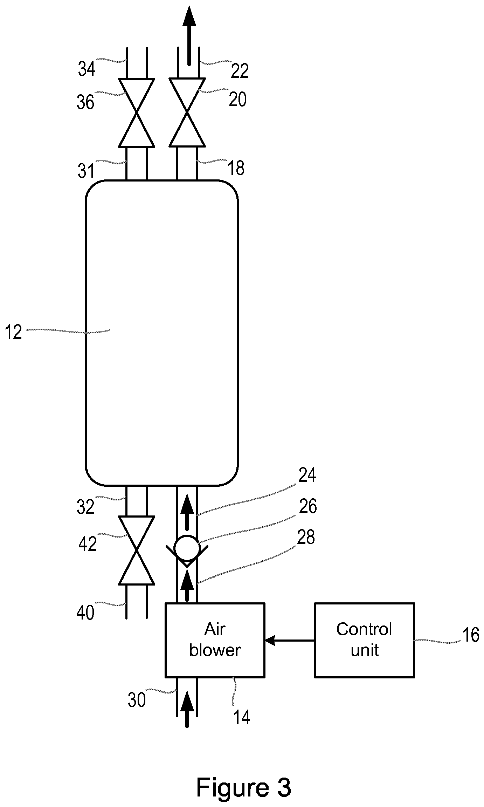

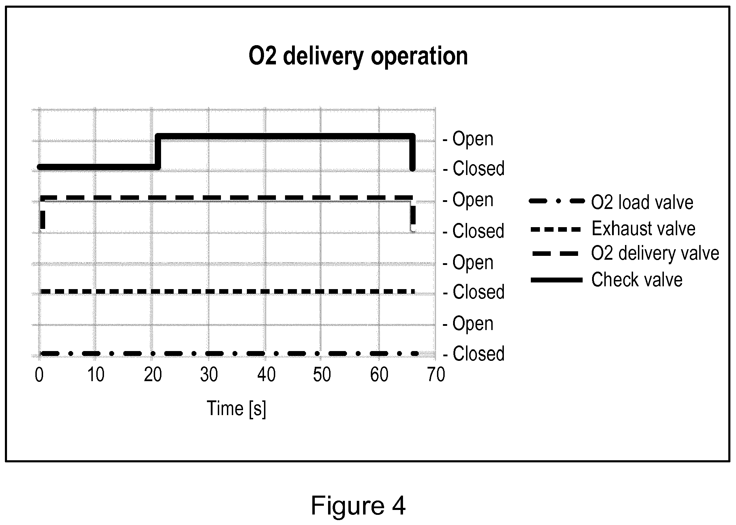

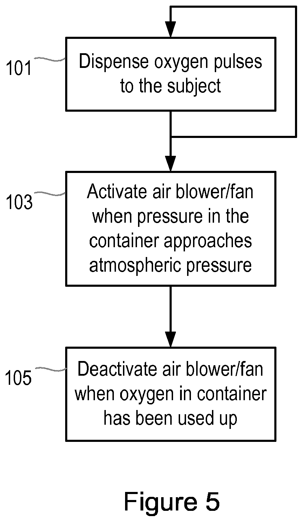

Figures 3, 4 and 5 illustrate the operation of the handheld device 4 of Figure 2 when oxygen-enriched air is to be delivered to the subject 8 according to an aspect of the invention. Figure 3 shows the handheld device 4 of Figure 2 with the direction of the gas flows shown, Figure 4 is a graph illustrating the operation of the valves 20, 26, 36 and 42 (although it will be appreciated that the specific timing of the opening and closing of the valves shown by the x-axis of the graph is merely exemplary and not limiting), and Figure 5 is a flow chart illustrating the method of operating the handheld device 4.

Initially, when the handheld device 4 is ready for use, the container 12 in the handheld device 4 is fully 'charged' with oxygen-enriched air (i.e. air with oxygen content greater than 21%) to a pressure that is higher than the air pressure around the handheld device (i.e. higher than atmospheric pressure). All valves 20, 26, 36 and 42 are closed and the air blower 14 is deactivated.

Preferably the container 12 is charged with gas that has an oxygen content of at least 40%, more preferably the container 12 is charged with gas that has an oxygen content of at least 60%, even more preferably the container 12 is charged with gas that has an oxygen content of at least 80%, and even more preferably the container 12 is charged with gas that has an oxygen content of at least 90%, for example 92%. Preferably the gas in the container 12 is at a pressure of at least 1.2 atmospheres, more preferably the gas in the container 12 is at a pressure of at least 1.5 atmospheres, and even more preferably the gas in the container 12 is at a pressure of at least 1.8 atmospheres. It has been found that the higher the initial pressure in the container 12, the better the performance of the handheld device 4 (for example in terms of the length of time that the handheld device 4 can be operated before requiring replenishment or recharge), although even with an initial oxygen concentration and/or initial pressure at the lower end of (or even below) the ranges provided above, performance of the handheld device 4 according to the invention is still considerably better than a stand-alone oxygen canister (i.e. a canister containing oxygen that does not include a fan or an adsorbent material) that contains gas with an equivalent oxygen concentration and starting pressure.

When a subject 8 requires supplemental oxygen, they can activate the handheld device 4 via a user interface or, in some embodiments, simply by inhaling through the subject interface 10. Activation of the handheld device 4 (which is shown as time 0 seconds in Figure 4) results in the delivery valve 20 being opened which allows a flow or pulse of oxygen-enriched air from the container 12 through the first conduit 22 and to the subject 8 (step 101 of Figure 5). It will be appreciated that although Figure 4 shows that the delivery valve 20 is open throughout the delivery operation (e.g. from 0 seconds to 66 seconds), in practice the delivery valve 20 is opened and closed in line with the subject's breathing during the delivery operation. The other valves 26, 36 and 42 remain closed.

As the subject 8 receives oxygen-enriched air from the container 12, the pressure in the container 12 will fall. In preparation for point where the pressure in the container 12 matches atmospheric pressure and the check valve 26 opens, the control unit 16 controls the air blower 14 to start operating shortly after the delivery valve 20 is first opened (this can be any time in the first 20 seconds in this example). This corresponds to step 103 in Figure 5. In other embodiments where the control unit 16 is monitoring the pressure in the container 12 (and/or otherwise controlling the operation of check valve 26), the control unit 16 can activate the air blower 14 when the monitored pressure is close to or at atmospheric pressure.

When the pressure in the container 12 matches atmospheric pressure and the check valve 26 opens, the air blower 14 will be pushing air (drawn from the outside of the handheld device 4) into the container 12 and through the adsorbent material 15. The adsorbent material 15 adsorbs specific gas or gases (e.g. nitrogen) from the air in order to increase the concentration of oxygen in the air supplied to the subject 8. The operation of the air blower 14 causes a small overpressure in the container (e.g. around 0.01-0.15 bar) which increases the rate at which air passes through the container 12 and to the subject 8 and also slightly increases the ability of the adsorbent material 15 to adsorb the specific gas or gases. The operation of the air blower 14 also acts to push the oxygen-enriched air initially stored in the container 12 out to the subject 8.

As the subject 8 continues to inhale oxygen-enriched air through the subject interface 10 and fresh air is blown into the container 12 by the air blower 14, the adsorbent material 15 gradually becomes saturated with the specific gas it is adsorbing (e.g. nitrogen).

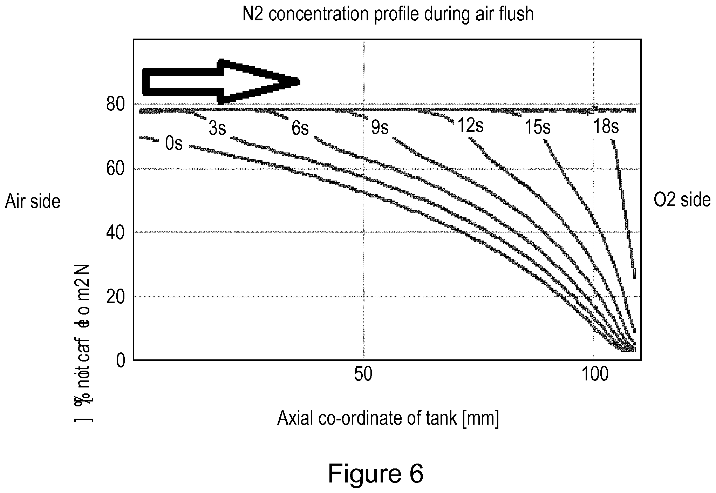

Figure 6 illustrates an exemplary nitrogen concentration profile during the operation of the handheld device 4, which is the axial profile yN2(z) of the N2 (nitrogen) mole fraction yN2. Neglecting Ar it can be stated that yO2(z) = 1-yN2(z). One interesting property of LiLSX zeolite is that high values of yN2 move faster than low values of yN2 when gas is streaming through the container 12. Figure 6 shows how a steep "front" (called "Mass Transfer Zone (MTZ)") is formed when fresh air is blown into the container 12. Thus, almost the full volume of the container 12 can be filled with air while the oxygen purity of the product (the gas output to the subject 12) stays high. Eventually the air front reaches the product side of the container 12 (where the first valve 20 is located) and the oxygen purity of the product drops suddenly (this is referred to as the "N2 breakthrough into the product").

When the adsorbent material 15 is completely saturated, the air output to the subject 8 will have the same oxygen content as the air blown into the container 12 by the air blower 14. At this stage the container 12 of the handheld device 4 requires recharging before it can be used again to provide supplemental oxygen to the subject 8. The control unit 16 therefore deactivates the air blower 14 (step 105). At this time the control unit 16 may also close or prevent further opening of the delivery valve 20 and/or close the check valve 26 (if it is controlled by the control unit 16). The control unit 16 can deactivate the blower 14 and close, or prevent further opening of, the delivery valve 20 (and optionally also the check valve 26 if it is under the control of the control unit 16) after the handheld device 4 has been used for a certain amount of time since the last full recharge of the device 4 or after a certain amount of time from the first inhalation (where the certain amount of time is set around the amount of time that the device 4 is expected to be able to provide supplemental oxygen), or after a certain number of inhalations by the subject 8 (where the certain number of inhalations is the total number of inhalations that the device 4 is expected to be able to provide supplemental oxygen). Alternatively, the control unit 16 can deactivate the blower 14 and close, or prevent further opening of, the delivery valve 20 (and check valve 26 as above) in response to detecting that the oxygen content of the container 12 or the gas output to the subject 8 is below or at a predetermined level (e.g. 21%), in which case an appropriate sensor can be provided to monitor the oxygen content of the product gas. As another alternative, the control unit 16 can deactivate the blower 14 and close, or prevent further opening of, the delivery valve 20 (and check valve 26 as above) in response to detecting an increase in the temperature of the gas flow to the subject 8. A temperature increase will occur when there is a 'breakthrough' of the air blown into the container 12 into the air flow to the subject 8, and this temperature increase will occur over a short period of time, for example of the order of 0.1 to 1 seconds. In the exemplary embodiment described herein, the temperature increase is of the order of 9K. In this embodiment, a temperature sensor can be coupled to the control unit 16 and provided in or at the first outlet 18 or in the first conduit 22 to measure this temperature change.

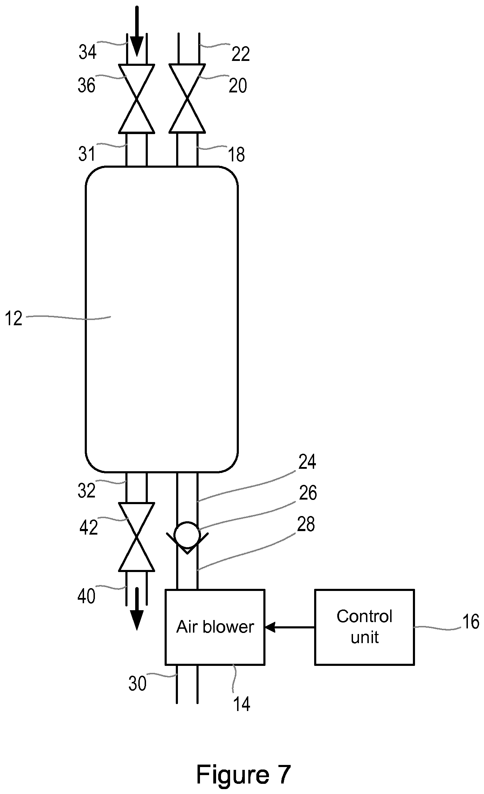

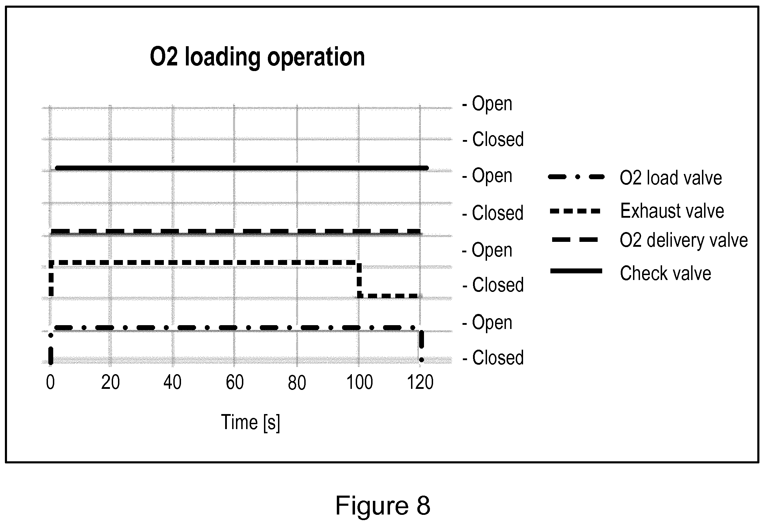

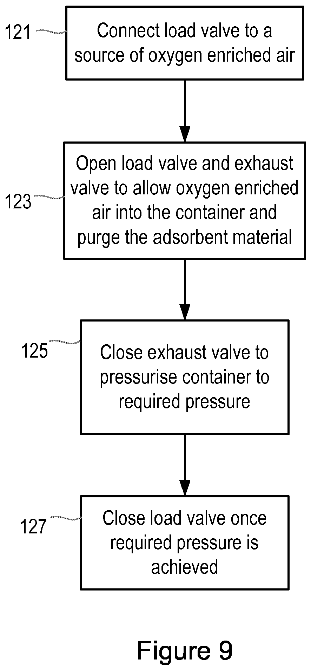

Figures 7, 8 and 9 illustrate the operation of the handheld device 4 of Figure 2 when the container 12 is to be recharged to enable oxygen-enriched air to again be delivered to the subject 8 according to an aspect of the invention. Figure 7 shows the handheld device 4 of Figure 2 with the direction of the gas flows shown, Figure 8 is a graph illustrating the operation of the valves 20, 26, 36 and 42 (although it will be appreciated that the specific timing of the opening and closing of the valves shown by the x-axis of the graph is merely exemplary and not limiting), and Figure 9 is a flow chart illustrating the method of operating the handheld device 4.

When the handheld device 4 requires recharging, the container 12 in the handheld device 4 will contain air with an oxygen content of around 21% (i.e. the same as the air around the handheld device 4) and the adsorbent material 15 will be saturated with the adsorbed gas (e.g. nitrogen). The recharging process according to this aspect of the invention purges the adsorbed gas from the adsorbent material 15 and the container 12. Preferably the recharging process also fills the container 12 with oxygen-enriched gas to a pressure that is preferably higher than atmospheric pressure. The process starts with all valves 20, 26, 36 and 42 being closed and the air blower 14 deactivated.

Before the recharging process can start, the handheld device 4 is connected to a source of oxygen or oxygen-enriched air, for example liquid oxygen or compressed gas or air with elevated oxygen levels, or an oxygen concentrator that operates according to the known pressure swing adsorption cycle principle and that supplies gas with elevated levels of oxygen (i.e. elevated above the normal oxygen content of air at sea level of around 21%). This is shown as step 121 in Figure 9. The source of oxygen or oxygen-enriched gas can be provided via a base unit 6 or directly to the handheld device 4. Preferably the oxygen-enriched gas has an oxygen content of at least 40%, more preferably an oxygen content of at least 60%, even more preferably an oxygen content of at least 80%, and even more preferably an oxygen content of at least 90%, for example 92%.

During the recharging process, the delivery valve 20 and check valve 26 remain closed. The load valve 36 and exhaust valve 42 are opened (step 123) by the control unit 16 (or optionally by a control unit in the base unit 6) at the start of the process to enable oxygen-enriched gas to flow into the container 12 and exhaust gases to flow out of the container 12 through the exhaust valve 42 and into the atmosphere.

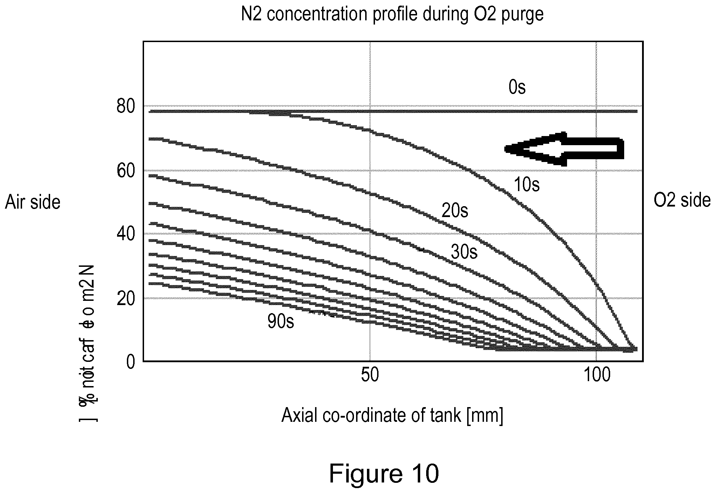

The flow of oxygen-enriched gas through the container purges the adsorbent material 15 causing the adsorbent material 15 to expel the adsorbed gas (e.g. nitrogen). This gas is then pushed out of the container 12 through the exhaust valve 42. Figure 10 illustrates an exemplary nitrogen concentration profile during the recharging of the handheld device 4, which is based on a simulation of the device 4. Figure 10 illustrates that high values of yN2 move faster than low values of yN2. Therefore, the high N2 concentration values will leave the container 12 quite early in the process, but the low N2 concentration values need much more time (and oxygen purge gas volume) to do so. This means that it is impossible to purge "all" of the nitrogen out of the adsorbent material 15. The actual "purge time" setting will be a compromise between the time required to reach the container's storage capacity and the oxygen purge volume required.

The flow of oxygen-enriched gas continues regardless of the concentration of the gas to be adsorbed in the container 12. This is in contrast to an oxygen concentrator, where purging is stopped when the concentration of the gas to be adsorbed goes below a threshold amount (e.g. below 78%) since it is important not to waste oxygen and thus ensure efficient oxygen concentration operation. Thus, in an oxygen concentrator, the purge duration is limited (typically to less than 20 seconds) to ensure this efficient operation (although this 'efficient' operation means that the adsorbent material is not fully purged of nitrogen). In contrast, in the handheld device 4 according to the invention, purging is performed in order to get the adsorbent material 15 in the container 12 is as clean as possible, and it does not matter that some oxygen provided in the purging or recharging phase is wasted (i.e. not stored in the container 12). In the exemplary embodiment described above, the purging step can last around 100 seconds.

Once the adsorbent material 15 is cleaned, the exhaust valve 42 is closed (as shown in Figure 8 and step 125 of Figure 9) and the flow of oxygen-enriched gas into the container 12 continues in order to pressurize the container 12 to the required initial pressure (e.g. 1.8 atmospheres in some embodiments).

Once the required initial pressure is reached, the load valve 36 is closed and the recharging process is completed (step 127). The device 4 is now ready for use by the subject 8.

An illustration of the performance of the handheld device 4 according to the invention is provided here for the exemplary embodiment above where the container 12 has a generally cylindrical shape, a length of around 0.11 meters, a diameter of around 0.04 meters and contains about 0.094 kg of LiLSX pellets. With the container 12 being charged with air enriched to 92% oxygen and to a pressure of 1.8 atmospheres, the handheld device 4 is able to deliver 21 pulses of oxygen-enriched air with a pulse volume for the subject 8 of 46 ml, which means that the handheld device 4 can be used continuously for around 65 seconds before requiring a recharge. For comparison, a handheld device 4 having a container charged to the same oxygen concentration and pressure but that does not include an adsorbent material 15 or an air blower 14 would provide just two 46 ml pulses of oxygen-enriched air, and allow the device to be used continuously for only around 6 seconds before requiring recharging.

Thus the use of a container 12 with an adsorbent material 15 and a fan 14 to blow additional air through the container 12 during use provides particularly strong performance compared to a standard container or canister of oxygen. This performance is caused in part by the oxygen contained in the air flow from the fan 14 being almost entirely added to the 92% (or other concentration) oxygen stream output to the subject 8 when the subject 8 inhales, and also by the adsorbent material 15 (e.g. zeolite sieve bed) being cooled by around 13 Kelvin (in the above example) due to desorption of the specific gas (e.g. nitrogen) during the oxygen purging/recharging process. This cooling increases the capacity of the adsorbent material to adsorb the specific gas by around 20%.

In the following four scenarios are considered for the above exemplary container 12 and initial oxygen content and pressure. The first scenario corresponds to a container 12 with no adsorbent material 15 or fan 14, the second to a container 12 with a fan 14 but no adsorbent material, the third to a container 12 with adsorbent material 15 but no fan 14 and the fourth to a container 12 as shown in Figure 2 with an adsorbent material 15 and a fan 14. In the first scenario, 101 ml of oxygen is output from the container 12 as it is depressurized from 1.8 atmospheres to 1 atmosphere. In the second scenario, in an ideal case, the oxygen remaining in the container 12 after depressurization to 1 atmosphere, approximately 126ml, is expelled by the air blown into the container 12 by the fan 14, which gives a total output of 227 ml. In the third scenario, there is never any air inside the container 12, only oxygen at 92% purity and at different pressures, which means that the temperature of the adsorbent material 15 (zeolite) hardly varies (only about by 2.4K). The presence of the adsorbent material 15 increases the oxygen capacity of the container 12 by a factor of about 2.85, which means that the amount of oxygen in the tank at 1.8 atmospheres is 647 ml (plus 196ml of nitrogen). This leads to 374ml of oxygen and 185 ml of nitrogen remaining in the container 12 when depressurized to 1 atmosphere. Therefore, 273 ml of oxygen is delivered. In the fourth scenario, the "empty" state is now the air-filled container at 1 atmosphere (which is 78 ml of oxygen plus 1.79 L of nitrogen). Purging the container 12 for 99 seconds with 1 standard liter per minute (slpm) of oxygen at 1 atmosphere and pressurized within 21 seconds to 1.8 atmospheres gives total gas amounts 743 ml of oxygen plus 550 ml nitrogen (which is higher than in the third scenario due to the temperature of the adsorbent material 15 decreasing by 13K due to nitrogen desorption during the purging with oxygen). The total oxygen output is now very high, because the 1.64L of air blown into the container 12 during operation also contains 344 ml of oxygen - and only a fraction of this oxygen remains in the container (around 78 ml). Therefore, in the fourth scenario, the total amount of oxygen delivered is 743 ml + 344 ml -78 ml = 1009ml.

In the above embodiments, the handheld device 4 is recharged by coupling the device 4 to a source of oxygen-enriched gas or to a base unit 6 that has a source of oxygen-enriched gas. In some embodiments, it may be possible to detach and remove the container 12 from the handheld device 4 to enable it to be recharged separately (for example in a base unit 6 or by coupling it to a source of oxygen-enriched gas). This embodiment can therefore allow a discharged container 12 to be swapped out for a previously-recharged container 12 and allow the device 4 to continue to be used straight away. In this embodiment, it will be appreciated that the container 12 will be configured so that when it is removed from the device 4 the inlets 18, 31 and outlets 24, 32 are closed to prevent air entering or leaving the container 12 (which is particularly important when the container 12 has been recharged). This may be achieved by configuring the valves 20, 26, 32 and 36 so that they are part of the removable container 12, or by providing additional valves on the container 12.

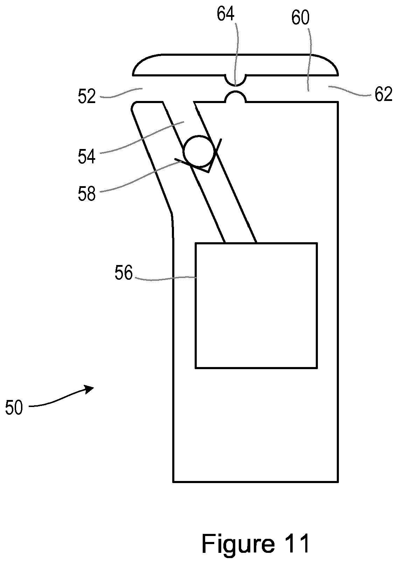

Figure 11 illustrates a handheld device 50 according to another or further aspect of the invention. In this aspect, the handheld device 50 is configured to provide supplemental oxygen to the subject 8 when they inhale, and to provide increased pressure or resistance to the subject 8 when they exhale. This handheld device 50 can be as described and shown in any of the above embodiments, and further comprise some means for applying a positive air pressure to the subject 8 when the subject 8 exhales through the subject interface (e.g. subject interface 52 in Figure 11). In alternative embodiments, the handheld device 50 can comprise a simple oxygen canister that does not include an adsorbent material or fan as described above, and the device 50 includes some means for applying a positive air pressure to the subject 8 when the subject 8 exhales through subject interface 52.

The illustrated handheld device 50 comprises a subject interface 52 through which the subject inhales and exhales, a first conduit 54 that couples the subject interface 52 to a container 56 that holds oxygen-enriched air (which can be as shown in Figure 2 above and further comprise an adsorbent material and/or a fan or air blower), a one-way valve or check valve 58 that prevents exhaled breath from entering the container 56 and a second conduit 60 that couples the subject interface 52 to an outlet (exhalation port) 62 through which exhaled breath passes. According to this aspect, the second conduit 60 includes some means 64 for applying a positive air pressure to the subject when the subject exhales. In some embodiments the means 64 is a partial obstruction or restriction in the second conduit 60 through which the subject exhales (e.g. a narrowing of the conduit 60 or other constriction). In some embodiments the means 64 can be implemented as a passive flow restriction in an exhalation port 62. In other embodiments, the means 64 actively provides positive pressure to the subject as they exhale. Those skilled in the art will be aware of ways in which positive air pressure can be actively provided to the subject using a handheld device, and thus those ways will not be described in further detail herein.

In some embodiments, the handheld device 4 or another part of the system 2 may comprise a sensor for measuring the oxygen levels in the subject 8. Such a sensor can be a sensor that measures the peripheral capillary oxygen saturation (SpO2).

In other or further embodiments, the handheld device 4 or system 2 can be part of a larger 'ecosystem' of devices, such as any one or more of a smart phone interface/application, smart sensors and various subject interfaces. As noted above, one sensor that can be used with the handheld device 4 is an SpO2 sensor for measuring peripheral oxygenation levels, for example at the fingertip or at the ear. In some embodiments the SpO2 sensor and/or other type of sensor is incorporated into a wrist watch or similar device to allow SpO2 to be measured during activity and/or exercise in order to identify possible exercise-induced desaturation events (e.g. oxygen levels below 90%). In these embodiments, the smart phone can be used to provide feedback or instructions to the subject 8 to supplement their oxygen levels in the event that the level is below the threshold.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other control unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A handheld device (4) for providing supplemental oxygen to a subject (8), the handheld device being designed to be easily held by the subject and readily portable for the subject, the device (4) comprising:
a housing (11);
a subject interface (10) through which the subject (8) can inhale;
a container (12) that has:
a first outlet (18) connected to the subject interface to allow gas with an elevated oxygen level stored in the container (12) to be inhaled by the subject (8),
a first inlet (24) for receiving air,
a second inlet (31) for receiving a purge gas;
a second outlet (32) for exhausting waste gas; and
a material (15) for removing a specific gas from air passing through the container (12) to increase the oxygen content of the air passing through the container (12);
an air blower (14) that is connected to the first inlet (24) of the container (12) and that is configured to supply air into the container (12) as the subject (8) uses the device (4); and
a first valve (20) located between the container (12) and the subject interface (10) for enabling a flow of supplemental oxygen to the subject (8),
wherein the fluid outlet (18) is connected to the subject interface via the first valve (20) and a first conduit (22),
wherein the housing has an air inlet (30) through which the air blower (14) can draw air into the device, and
wherein the housing has a supply port (38) for receiving the purge gas,
wherein the housing has an exhaust port (44) for exhausting the waste gas.

2. A device (4) as claimed in claim 1, wherein said device further comprise a control unit (16) for controlling the operation of the device (4).

3. A device (4) as claimed in claim 2, wherein the container (12) is configured to store gas with an elevated oxygen level at a pressure above atmospheric pressure.

4. A device (4) as claimed in claim 3, wherein the air blower (14) is configured to be activated by the control unit (16) to supply air into the container (12) when the pressure of the gas with an elevated oxygen level stored in the container (12) is at or close to atmospheric pressure.

5. A device (4) as claimed in claim 4, wherein the air blower (14) is configured to be deactivated by the control unit (16) when any one of the following conditions is satisfied:
(i) when the material (15) is saturated with the specific gas;
(ii) after a predetermined time from a first inhalation by the subject (8);
(iii) after the subject (8) has used the device (4) for a predetermined time from the last recharge of the device (4);
(iv) after a predetermined number of inhalations by the subject (8);
(v) when there is an increase in the temperature of the gas supplied to the subject (8); or
(vi) when the oxygen content of the gas supplied to the subject (8) is below a threshold.

6. A device (4) as claimed in any preceding claim, wherein the first inlet (24) and the first outlet (18) are arranged with respect to each other such that air blown into the container (12) through the first inlet (24) by the air blower (14) pushes out gas with an elevated oxygen level stored in the container (12) through the first outlet (18).

7. A device (4) as claimed in any preceding claim, the device (4) further comprising a check valve (26) positioned between the air blower (14) and the first inlet (24), the check valve (26) being configured to close when the pressure of the gas with an elevated oxygen level stored in the container (12) is above atmospheric pressure in order to prevent gas in the container (12) from flowing through the air blower (14) when the air blower (14) is deactivated, and being configured to open when the pressure of the gas with an elevated oxygen level stored in the container (12) is at or below atmospheric pressure in order to allow air to be supplied into the container (12) by the air blower (14); the second inlet (31) is configured to receive gas with an elevated oxygen level during a recharging process, and the second outlet (32) is configured to exhaust waste gas during the recharging process; the device (4) further comprising a load valve (36) connected to the second inlet (31) that is configured to be opened at the start of a recharging process to allow gas with an elevated oxygen level to be supplied into the container (12) in order to purge the material of the specific gas, and an exhaust valve (42) connected to the second outlet (32) that is configured to be opened at the start of the recharging process to allow waste gas to exit the container (12).

8. A device (4) as claimed in claim 7, wherein the exhaust valve (42) is configured to be closed during the recharging process while gas with an elevated oxygen level is supplied into the container (12) through the load valve (36) and second inlet (31) in order to store gas with an elevated oxygen level in the container (12).

9. A device (4) as claimed in any preceding claim, the device (4) further comprising:
means (64) for applying a positive air pressure to the subject (8) when the subject (8) exhales through the subject interface (10).

10. A supplemental oxygen system (2) comprising:
a handheld device (4) as claimed in any of claims 1-9; and
a source (6) of gas with an elevated oxygen level that can be selectively coupled to the device (4) in order to recharge the device with gas with an elevated oxygen level and/or to purge the material of the specific gas.

## Patentansprüche

1. Tragbare Vorrichtung (4) zur Bereitstellung von zusätzlichem Sauerstoff für ein Subjekt (8), wobei die tragbare Vorrichtung gestaltet ist, leicht vom Subjekt gehalten und leicht für das Subjekt tragbar zu sein, die Vorrichtung (4) umfassend:
ein Gehäuse (11);
eine Subjektschnittstelle (10), durch die das Subjekt (8) einatmen kann;
einen Behälter (12), der aufweist:
einen ersten Auslass (18), der mit der Subjektschnittstelle verbunden ist, sodass Gas mit erhöhtem Sauerstoffgehalt, das im Behälter (12) gespeichert ist, vom Subjekt (8) eingeatmet werden kann,
einen ersten Einlass (24) zum Aufnehmen von Luft,
einen zweiten Einlass (31) zum Aufnehmen eines Spülgases;
einen zweiten Auslass (32) zum Abgeben von Abgas; und
ein Material (15) zum Entfernen eines spezifischen Gases aus Luft, die durch den Behälter (12) geht, um den Sauerstoffgehalt der Luft, die durch den Behälter (12) geht, zu erhöhen;
ein Luftgebläse (14), das mit dem ersten Einlass (24) des Behälters (12) verbunden ist und gestaltet ist, Luft in den Behälter (12) zu leiten, wenn das Subjekt (8) die Vorrichtung (4) verwendet; und
ein erstes Ventil (20), das zwischen dem Behälter (12) und der Subjektschnittstelle (10) gelegen ist, um einen Strom an zusätzlichem Sauerstoff zum Subjekt (8) zu ermöglichen,
wobei der Fluidauslass (18) mit der Subjektschnittstelle über das erste Ventil (20) und eine erste Leitung (22) verbunden ist,
wobei das Gehäuse einen Lufteinlass (30) aufweist, durch den das Luftgebläse (14) Luft in die Vorrichtung ziehen kann; und
wobei das Gehäuse einen Zuleitungsanschluss (38) zum Aufnehmen des Spülgases aufweist,
wobei das Gehäuse einen Abgabeanschluss (44) zum Abgeben des Abgases aufweist.

2. Vorrichtung (4) nach Anspruch 1, wobei die Vorrichtung weiter eine Steuereinheit (16) zum Steuern des Betriebs der Vorrichtung (4) umfasst.

3. Vorrichtung (4) nach Anspruch 2, wobei der Behälter (12) gestaltet ist, Gas mit einem erhöhten Sauerstoffgehalt bei einem Druck über Atmosphärendruck zu speichern.

4. Vorrichtung (4) nach Anspruch 3, wobei das Luftgebläse (14) gestaltet ist, durch die Steuereinheit (16) aktiviert zu werden, um Luft in den Behälter (12) zu leiten, wenn der Druck des Gases mit einem erhöhten Sauerstoffgehalt, das im Behälter (12) gespeichert ist, bei oder nahe Atmosphärendruck ist.

5. Vorrichtung (4) nach Anspruch 4, wobei das Luftgebläse (14) gestaltet ist, durch die Steuereinheit (16) deaktiviert zu werden, wenn eine der folgenden Bedingungen erfüllt ist:
(i) wenn das Material (15) mit dem spezifischen Gas gesättigt ist;
(ii) nach einer vorbestimmten Zeit nach einen ersten Einatmen durch das Subjekt (8);
(iii) nachdem das Subjekt (8) die Vorrichtung (4) über eine vorbestimmte Zeit seit der letzten Aufladung der Vorrichtung (4) verwendet hat;
(iv) nach einer vorbestimmten Anzahl von Einatmungsvorgängen durch das Subjekt (8);
(v) wenn eine Erhöhung in der Temperatur des Gases vorliegt, das dem Subjekt (8) zugeleitet wird; oder
(vi) wenn der Sauerstoffgehalt des Gases, das dem Subjekt (8) zugeleitet wird, unter einem Schwellenwert ist.

6. Vorrichtung (4) nach einem der vorstehenden Ansprüche, wobei der erste Einlass (24) und der erste Auslass (18) in Bezug zueinander so angeordnet sind, dass Luft, die durch das Luftgebläse (14) durch den ersten Einlass (24) in den Behälter (12) geblasen wird, Gas mit einem erhöhten Sauerstoffgehalt, das im Behälter (12) gespeichert ist, durch den ersten Auslass (18) hinausschiebt.

7. Vorrichtung (4) nach einem der vorstehenden Ansprüche, wobei die Vorrichtung (4) weiter ein Rückschlagventil (26) umfasst, das zwischen dem Luftgebläse (14) und dem ersten Einlass (24) positioniert ist, wobei das Rückschlagventil (26) gestaltet ist, sich zu schließen, wenn der Druck des Gases mit einem erhöhten Sauerstoffgehalt, das im Behälter (12) gespeichert ist, über Atmosphärendruck liegt, um zu verhindern, das Gas im Behälter (12) durch das Luftgebläse (14) strömt, wenn das Luftgebläse (14) deaktiviert ist, und gestaltet ist, sich zu öffnen, wenn der Druck des Gases mit einem erhöhten Sauerstoffgehalt, das im Behälter (12) gespeichert ist, bei oder unter Atmosphärendruck liegt, um zu ermöglichen, dass Luft durch das Luftgebläse (14) in den Behälter (12) geleitet wird;
der zweite Einlass (31) gestaltet ist, Gas mit einem erhöhten Sauerstoffgehalt während eines Aufladungsprozesses aufzunehmen, und der zweite Auslass (32) gestaltet ist, Abgas während des Aufladungsprozesses abzugeben; wobei die Vorrichtung (4) weiter ein Ladeventil (36) umfasst, das mit dem zweiten Einlass (31) verbunden ist, der gestaltet ist, bei Beginn eines Aufladungsprozesses geöffnet zu werden, um zu ermöglichen, dass Gas mit einem erhöhten Sauerstoffgehalt in den Behälter (12) geleitet wird, um das Material des spezifischen Gases auszuspülen, sowie ein Abgabeventil (42), das mit dem zweiten Auslass (32) verbunden ist, das gestaltet ist, bei Beginn des Aufladungsprozesses geöffnet zu werden, um zu ermöglichen, dass Abgas aus dem Behälter (12) austritt.

8. Vorrichtung (4) nach Anspruch 7, wobei das Abgabeventil (42) gestaltet ist, während des Aufladungsprozesses geschlossen zu werden, während Gas mit einem erhöhten Sauerstoffgehalt durch das Ladeventil (36) und den zweiten Einlass (31) in den Behälter (12) geleitet wird, um Gas mit einem erhöhten Sauerstoffgehalt im Behälter (12) zu speichern.

9. Vorrichtung (4) nach einem der vorstehenden Ansprüche, wobei die Vorrichtung (4) weiter umfasst:
Mittel (64) zum Anwenden eines Luftüberdrucks bei dem Subjekt (8), wenn das Subjekt (8) durch die Subjektschnittstelle (10) ausatmet.

10. Zusatzsauerstoffsystem (2), umfassend:
eine tragbare Vorrichtung (4) nach einem der Ansprüche 1-9; und
eine Quelle (6) von Gas mit einem erhöhten Sauerstoffgehalt, die selektiv an die Vorrichtung (4) gekoppelt werden kann, um die Vorrichtung mit Gas mit einem erhöhten Sauerstoffgehalt aufzuladen und/oder das Material des spezifischen Gases auszuspülen.

## Revendications

1. Dispositif portatif (4) pour fournir de l'oxygène d'appoint à un sujet (8), le dispositif portatif étant conçu pour être aisément maintenu par le sujet et aisément portable pour le sujet, le dispositif (4) comprenant :
un boîtier (11) ;
une interface sujet (10) à travers laquelle le sujet (8) peut inhaler ;
un récipient (12) qui a :
une première sortie (18) raccordée à l'interface sujet pour permettre l'inhalation par le sujet (8) de gaz avec un niveau d'oxygène élevé stocké dans le récipient (12),
une première entrée (24) pour recevoir de l'air,
une seconde entrée (31) pour recevoir un gaz de purge ;
une seconde sortie (32) pour évacuer du gaz résiduaire ; et
un matériau (15) pour éliminer un gaz spécifique de l'air passant à travers le récipient (12) afin d'augmenter la teneur en oxygène de l'air passant à travers le récipient (12) ;
une soufflerie d'air (14) qui est raccordée à la première entrée (24) du récipient (12) et qui est configurée pour fournir de l'air dans le récipient (12) lorsque le sujet (8) utilise le dispositif (4) ; et
une première soupape (20) située entre le récipient (12) et l'interface sujet (10) pour permettre un écoulement d'oxygène d'appoint vers le sujet (8),
dans lequel la sortie de fluide (18) est raccordée à l'interface sujet via la première soupape (20) et un premier conduit (22),
dans lequel le boîtier a une entrée d'air (30) à travers laquelle la soufflerie d'air (14) peut aspirer de l'air dans le dispositif et
dans lequel le boîtier a un orifice d'alimentation (38) pour recevoir le gaz de purge,
dans lequel le boîtier a un orifice d'échappement (44) pour évacuer le gaz résiduaire.

2. Dispositif (4) selon la revendication 1, dans lequel ledit dispositif comprend en outre une unité de commande (16) pour commander le fonctionnement du dispositif (4).

3. Dispositif (4) selon la revendication 2, dans lequel le récipient (12) est configuré pour stocker du gaz avec un niveau d'oxygène élevé à une pression supérieure à la pression atmosphérique.

4. Dispositif (4) selon la revendication 3, dans lequel la soufflerie d'air (14) est configurée pour être activée par l'unité de commande (16) afin de fournir de l'air dans le récipient (12) lorsque la pression du gaz avec une teneur en oxygène élevée stocké dans le récipient (12) se trouve à la pression atmosphérique ou près de celle-ci.

5. Dispositif (4) selon la revendication 4, dans lequel la soufflerie d'air (14) est configurée pour être désactivée par l'unité de commande (16) lorsque l'une quelconque des conditions suivantes est satisfaite :
(i) lorsque le matériau (15) est saturé du gaz spécifique ;
(ii) après une période prédéterminée depuis une première inhalation par le sujet (8) ;
(iii) après que le sujet (8) a utilisé le dispositif (4) pendant une période prédéterminée depuis la dernière recharge du dispositif (4) ;
(iv) après un nombre prédéterminé d'inhalations par le sujet (8) ;
(v) lorsqu'il y a une augmentation de la température du gaz fourni au sujet (8) ; ou
(vi) lorsque la teneur en oxygène du gaz fourni au sujet (8) se situe en dessous d'un seuil.

6. Dispositif (4) selon l'une quelconque des revendications précédentes, dans lequel la première entrée (24) et la première sortie (18) sont agencées l'une par rapport à l'autre de sorte que l'air soufflé dans le récipient (12) à travers la première entrée (24) par la soufflerie d'air (14) éjecte le gaz avec un niveau d'oxygène élevé stocké dans le récipient (12) à travers la première sortie (18).

7. Dispositif (4) selon l'une quelconque des revendications précédentes, le dispositif (4) comprenant en outre une soupape de retenue (26) positionnée entre la soufflerie d'air (14) et la première entrée (24), la soupape de retenue (26) étant configurée pour se fermer lorsque la pression du gaz d'un niveau d'oxygène élevé stocké dans le récipient (12) se situe au-dessus de la pression atmosphérique afin d'empêcher le gaz du récipient (12) de s'écouler à travers la soufflerie d'air (14) lorsque la soufflerie d'air (14) est désactivée et étant configuré pour s'ouvrir lorsque la pression du gaz d'un niveau d'oxygène élevé stocké dans le récipient (12) se situe à la pression atmosphérique ou en dessous de celle-ci afin de permettre l'alimentation en air dans le récipient (12) par la soufflerie d'air (14) ;
la seconde entrée (31) est configurée pour recevoir du gaz d'un niveau d'oxygène élevé au cours d'un processus de recharge et la seconde sortie (32) est configurée pour décharger du gaz résiduaire au cours du procédé de recharge, le dispositif (4) comprenant en outre une soupape de charge (36) raccordée à la seconde entrée (31) qui est configurée pour être ouverte au début du processus de recharge afin de permettre de fournir du gaz d'un niveau d'oxygène élevé dans le récipient (12) afin de purger le matériau du gaz spécifique, et une soupape d'échappement (42) raccordée à la seconde sortie (32) qui est configurée pour être ouverte au départ du processus de recharge afin de permettre au gaz résiduaire de sortir du récipient (12).

8. Dispositif (4) selon la revendication 7, dans lequel la soupape d'échappement (42) est configurée pour être fermée au cours du processus de recharge tandis que le gaz d'un niveau d'oxygène élevé est fourni au récipient (12) à travers la soupape de charge (36) et la seconde entrée (31) afin de stocker du gaz d'un niveau d'oxygène élevé dans le récipient (12).

9. Dispositif (4) selon l'une quelconque des revendications précédentes, le dispositif (4) comprenant en outre :
des moyens (64) pour appliquer une pression d'air positive au sujet (8) lorsque le sujet (8) exhale à travers l'interface sujet (10).

10. Système d'oxygène d'appoint (2) comprenant :
un dispositif portable (4) selon l'une quelconque des revendications 1 à 9 ; et
une source (6) de gaz d'un niveau d'oxygène élevé qui peut être sélectivement couplée au dispositif (4) afin de recharger le dispositif en gaz d'un niveau d'oxygène élevé et/ou de purger le matériau du gaz spécifique.
